# EUROPÄISCHE PATENTANMELDUNG

(11) **EP 3 338 721 A1**
(43) Veröffentlichungstag der Anmeldung: **27.06.2018**
(21) Anmeldenummer: 18156556.5
(22) Anmeldetag: 01.12.2011
(51) Int. Cl.: A61B 17/70, A61B 17/86, A61B 17/80

(54) **WINKELVARIABLE KNOCHENSCHRAUBEN-FIXATIONSANORDNUNG**

(30) Priorität: 01.12.2010 EP 10015172
(62) Teilanmeldung aus: 11788861.0
(71) Anmelder: FACET-LINK Inc., Rockaway NJ 07866 (US)
(72) Erfinder: LINK, Helmut D., 22397 Hamburg (DE); JENSEN, Harm-Iven, 24214 Noer (DE)
(74) Vertreter: Glawe, Delfs, Moll

(57) **Zusammenfassung**

Die Erfindung betrifft eine Fixationsanordnung umfassend einen Halter (2') und eine Knochenschraube (1') aufnehmende Spannhülse (4'), wobei die Knochenschraube (1') einen Schaft (10') mit einem vorderen Gewinde (11') und einen Kopf (12') am hinteren Ende aufweist, wobei der Halter (3') mit einer Durchgangsbohrung (30') und einem Aufnahmesitz (32') versehen ist, in dem die Spannhülse (4') schwenkbeweglich gelagert ist, wobei der Schaft (10') vor dem Kopf (12') in einem hinteren Bereich eine Verdickung (15') aufweist, deren Weite zum Kopf (12') hin zunimmt, und die Weite der Spannhülse (4') größer ist als die Weite des Schafts (10') unmittelbar vor der Verdickung (15') und kleiner als die größte Weite der Verdickung (15'). Bei dem Einschrauben weitet die Verdickung (15) die Spannhülse (4'), so dass sie mit ihrem Mantel gegen die Wandung des Aufnahmesitzes (32') gepresst wird. Damit wird eine winkelstabile Verpressung bei an sich beliebiger Ausrichtung der Knochenschraube (1') erreicht.

## Beschreibung

Die Erfindung betrifft eine Fixationsanordnung zur Befestigung mittels einer Knochenschraube. Die Fixationsanordnung dient insbesondere zur Verbindung mit einem Haltestab für ein Implantat, kann aber auch eigenständig verwendet sein.

Zur einerseits lösbaren aber andererseits hinreichend festen Befestigung von Implantaten werden häufig Schrauben verwendet. Je nach Anwendungsfall ist hierbei eine winkelstarre Positionierung zwischen dem Implantat und der Knochenschraube ausreichend, in anderen Fällen ist aber eine winkelvariable (polyaxiale) Anordnung der Knochenschrauben erforderlich. Eine derartige polyaxial gelagerte Knochenschraube für eine Fixationsanordnung ist in ihren Grundzügen aus der EP 0 614 649 B1 bekannt. Die Knochenschraube ist in einer Haltehülse geführt, welche zur Sicherung eine aufschraubbare Mutter aufweist. Zur Befestigung eines Stabs von einem Implantat ist an der Haltehülse eine Querbohrung vorgesehen, durch welche der Stab in einen Zwischenbereich zwischen der Mutter und der Knochenschraube eingeführt werden kann. Durch Anziehen der Mutter wird der Stab mit der Haltehülse verspannt. Bei hohen Anzugskräften kann es zur Verwindung der Haltehülse kommen, wodurch die Befestigungssicherheit gefährdet ist.

Bei einer weiterentwickelten Konstruktion, wie sie aus der US 7,641,674 B2 bekannt ist, wirkt eine am hinteren Ende der Haltehülse eingeschraubte Druckschraube über den durch eine Querbohrung gesteckten Haltestab des Implantats auf ein Spannelement, welches an seiner vorderen Seite eine kalottenförmige Ausnehmung zur Aufnahme eines kugelförmig verdickten Schraubenkopfs der Knochenschraube aufweist. Das Spannelement ist längsverschieblich und ist unter der Kraft der Druckschraube gegen einen am vorderen Ende der Haltehülse angeordneten Sprengring gedrückt. So bildet er ein Gegenlager für die von der Druckschraube aufgebrachte Druckkraft. Diese Bauweise begrenzt die aufzubringenden Druckkräfte, und damit die zur Erreichung einer Winkelstabilität erforderlichen Klemmkräfte.

Der Erfindung liegt die Aufgabe zugrunde, ausgehend von dem zuletzt genannten Stand der Technik eine verbesserte Fixationsanordnung zu schaffen, die eine bessere Winkelfixierung erreicht.

Die erfindungsgemäße Lösung liegt in einer Fixationsanordnung mit den Merkmalen der unabhängigen Ansprüche. Vorteilhafte Weiterbildungen sind Gegenstand der abhängigen Ansprüche.

Bei einer Fixationsanordnung umfassend einen Halter und eine eine Knochenschraube aufnehmende Spannhülse, wobei die Knochenschraube einen Schaft mit einem vorderen Gewinde und einen Kopf am hinteren Ende aufweist, und der Halter eine Durchgangsbohrung mit einem Aufnahmesitz aufweist, in dem die Spannhülse schwenkbeweglich gelagert ist, ist erfindungsgemäß vorgesehen, dass der Schaft in seinem hinteren Bereich vorderseitig vom Kopf eine Verdickung aufweist, deren Weite zum Kopf hin zunimmt, und die Weite der Spannhülse größer ist als die Weite des Schafts unmittelbar vor der Verdickung und kleiner als die größte Weite der Verdickung.

Unter "vorne" wird die Richtung zur Spitze und unter "hinten" die Richtung zum Kopf der Knochenschraube verstanden.

Die von vorne nach hinten zunehmende Verdickung erreicht am Kopf ihre größte Weite, die größer ist als die lichte Weite der Spannhülse. Vorzugsweise erstreckt sich die Verdickung über eine Länge, die mindestens das Zweieinhalbfache der Länge der Spannhülse beträgt. Besonders bevorzugt ist es, wenn die Verdickung konisch ist, und zwar mit einem Konuswinkel von maximal 15 Grad, vorzugsweise maximal 5 Grad.

Unter schwenkbeweglich wird hierbei verstanden, dass die Achse der mittels der Spannhülse fixierten Knochenschraube einen frei einstellbaren Winkel zur Achse der Durchgangsbohrung in der Halterung einnehmen kann. Der Einstellbereich beträgt vorzugsweise mindestens 15 Grad in jeder Richtung um die Mittelachse der Durchgangsbohrung.

Die Erfindung beruht auf dem Gedanken durch eine Kombination zweier Maßnahmen für eine wesentliche Steigerung der die Schraube in ihrer Winkellage sichernden Kraft zu sorgen. Zum einen wird mit der Anordnung der Verdickung vor dem Kopf der Knochenschraube ein Herausrutschen der Knochenschraube aus der Spannhülse auch bei sehr hoher Kraftbelastung verhindert. So kann am Implantationsort die Knochenschraube angezogen werden, wobei sie sich axial nach vorne verschiebt. Zum anderen wird aufgrund dieser axialen Bewegung die Verdickung in die Spannhülse hinein bewegt und drückt gegen deren Innenwandung. Dadurch wird die Spannhülse mit ihrem vorzugsweise sphärischen Mantel elastisch aufgeweitet und verklemmt mit der vorzugsweise komplementär geformten, d. h. kugelkalottenförmigen Wandung des Aufnahmesitzes. Je nach Art der Verdickung ergibt sich damit eine beträchtliche Kraftübersetzung, bei einem Konuswinkel von 5 Grad über zehnfach. Damit kann effektiv eine Verpressung zwischen Spannhülse und Aufnahmesitz erreicht werden. Die Knochenschraube ist damit in der gewünschten Winkelstellung durch Preßsitz fixiert.

Meist wird die Verdickung einen glatten Mantel aufweisen. Dies ermöglicht ein reibungsarmes Anziehen der Knochenschraube und eine vereinfachte axiale Relativbewegung zur Spannhülse. Es kann aber auch vorgesehen sein, dass an der Verdickung ein Sekundärgewinde ausgebildet ist. Es weist vorzugsweise dieselbe Steigung wie das Gewinde auf dem Schaft auf. Besonders bevorzugt ist es, wenn das Sekundärgewinde mehrgängig ist. Das vereinfacht das Einschrauben besonders bei nur kurzem Gegengewinde. Das Gegengewinde kann gebildet sein in der Spannhülse, und zwar vorzugsweise an einer Randleiste, die an der vorzugsweise zylinderförmigen Innenwandung der Spannhülse umläuft. Die Radialleiste verhindert ein Auswandern der Knochenschraube, und das dort angeordnete Innengewinde erleichtert die Spreizung der Spannhülse und erhöht so weiter die Befestigungssicherheit.

Bei einer bevorzugten Ausführungsform ist der Mantel der Verdickung mit einem eigenen Gewinde (Sekundärgewinde) versehen. Das Sekundärgewinde ist vorzugsweise mit einer Steigung ausgebildet, die kleiner ist als die Steigung des Gewindes am Schaft, zweckmäßigerweise im Bereich zwischen 0,4 und 0,6. Das Sekundärgewinde ist also feiner als das Gewinde am Schaft und daher vorzugsweise eingängig ausgeführt. Eine mehrgängige Ausführung soll jedoch nicht ausgeschlossen sein. Vor dem Mantel, an dem das Sekundärgewinde ausgebildet ist, ist vorzugsweise ein konischer Abschnitt vorgesehen, über den die Weite von der Weite des Schafts ansteigt bis zur Weite des Mantels. Der Mantel selbst ist zweckmäßigerweise zylindrisch, jedoch ist dies nicht zwingen. Bevorzugterweise ist der Kopf konisch ausgeführt, und zwar derart, dass er an die Verdickung unmittelbar anschließt und sich nach hinten erweitert.

Es ist nicht unbedingt erforderlich, dass die Verdickung sich aus dem Schaft heraus gleichmäßig erhebt. Es kann auch vorgesehen sind, dass die Verdickung an ihrem vorderen Ende einen Bund bildet. Besonders zweckmäßig ist es, in diesem Fall die Verdickung mit dem Kopf der Knochenschraube baulich zu kombinieren, wobei dann der Außenmantel der Verdickung eine Konizität aufweist, die sich weiter vorzugsweise auch über den Kopf erstrecken kann.

Weiter kann vorgesehen sein, dass die Knochenschraube ein konisches Gewinde mit einem zum Kopf hin zunehmenden Kerndurchmesser aufweist. Mit einem solchen Gewinde kann eine gute Schneidwirkung im Knochen mit hoher Befestigungssicherheit einfacher Implantationstechnik erreicht werden.

Die Spannhülse ist vorzugsweise geschlitzt ausgeführt. Dies kann auf verschiedene Weise bewerkstelligt sein. So kann ein über die gesamte Länge der Spannhülses verlaufender durchgehender Schlitz vorgesehen sein. Damit ergibt eine gute Aufweitbarkeit, allerdings zulasten einer rotationssymmetrischen Kraftableitung an die Wandung des Aufnahmesitzes. Alternativ (oder auch zusätzlich) kann vorgesehen sein, dass mehrere einseitig offene Schlitze vorgesehen sind. Zweckmäßigerweise sind sie zur vorderen Seite hin offen.

Die Spannhülse kann mit einer komplexen Durchgangsöffnung versehen sein. Darunter wird verstanden, dass die Durchgangsöffnung eine konischen Abschnitt zum Bewirken der Spreizfunktion und einen Gewindeabschnitt aufweist, der vorzugsweise kurz (höchstens zwei Gewindeumdrehungen) ist und zum Spannen dient.

Bei einer alternativen Ausführungsform, die ggf. unabhängigen Schutz verdient, umfasst die Fixationsanordnung eine Schraube und einen Halter, wobei die Schraube einen Gewindeschaft und einen Kugelkopf aufweist, und der Halter eine Haltehülse, ein Spannelement und ein Druckelement umfasst, wobei die Haltehülse eine durchlaufende Innenbohrung mit einem vorderen Passsitz für das Spannelement und einem hinteren Innengewinde für das Druckelement versehen ist, und wobei das Spannelement ein Spannkäfig ist, und im eingesetzten Zustand der Kugelkopf über dessen Bereich größter Weite hinaus umschlossen ist und über eine konische Grenzfläche so mit dem Passsitz zusammenwirkt, dass der Spannkäfig bei einer axialen Verschiebung nach vorne zusammengedrückt und gegen den Kugelkopf verpresst ist.

Dies beruht auf dem Gedanken, durch eine Kombination zweier Maßnahmen für eine wesentliche Steigerung der die Schraube in ihrer Winkellage sichernden Kraft zu sorgen. Dies erfolgt zum einen dadurch, dass der Spannkäfig den Kugelkopf der Schraube über dem Äquator hinaus umschließt, und damit ein Herausrutschen oder herausgedrückt werden des Kugelkopfs auch bei sehr hoher Kraftbelastung ausschließt. Dies wird kombiniert mit der erfindungsgemäß vorgesehenen Konizität zwischen dem Spannkäfig und dessen Passsitz in der Haltehülse, wodurch sich unter Einwirkung der von dem Druckelement aufgebrachten Druckkraft eine Verengung des Spannkäfigs mit dem darin aufgenommenen Kugelkopf der Schraube ergibt. Der den Kugelkopf umschließende Mantel des Spannkäfigs wird dabei mit einer aufgrund von Keilwirkung erheblich verstärkten Kraft in den Kugelkopf gedrückt, so dass sich eine Verpressung ergibt. Dank dieser Verpressung kombiniert mit dem Schutz vor Herausrutschen auch bei der sehr hohen Kraftbeaufschlagung wird eine sehr stabile Winkelfixierung erreicht.

Um die vom Spannkäfig ausgeübten Presskräfte zu erhöhen, ist der Spannkäfig vorzugsweise an seinem Mantel mit zum vorderen Ende hin offenen Schlitzen versehen. Damit kann der Spannkäfig auch bei erheblicher Komprimierung die durch die Konizität erzeugten Presskräfte ungehindert auf den Kugelkopf übertragen, ohne dass es zu einer unerwünschten internen Verzwängung im Spannkäfig kommt.

Mit Vorteil wirkt das Druckelement auf einen Außenrand der Stirnseite des Spannkäfigs. Indem die Druckkraft auf den Außenrand und nicht auf das Zentrum des Spannkäfigs wirkt, erfolgt eine direkte Kraftüberleitung zu der konischen Grenzfläche zwischen Außenmantel des Spannkäfigs und dem Passsitz. Weiter wird damit vermieden, dass der Spannkäfig im Zentrum unter der Last der Druckkraft eingedrückt wird, wodurch auf den Kugelkopf eine ihn aus dem Spannkäfig ausschiebende Kraft erzeugt wird. Die Befestigungssicherheit wird damit erhöht.

Mit Vorteil weist der Spannkäfig an seinem vorderen Ende einen radial vorspringenden Rand auf. Dies ermöglicht eine Vormontage des Spannkäfigs in der Haltehülse derart, dass der Spannkäfig von vorne in die Haltehülse eingeschoben wird und - nach Passage der Verengung am vorderen Rand der Haltehülse - in seine Position schnappt, wobei der radial vorspringende Rand sich noch außerhalb der Verengung befindet und die Spannhülse an einem Auswandern nach hinten zum Gewinde hindert. In diese Einheit ist der Kugelkopf der Knochenschraube einpreßbar. Mit einer solchen Vormontage ist die Anwendbarkeit für den Chirurgen deutlich erleichtert, und außerdem ist das für den Patienten hohe Risiko aus einem möglichen Teileverlust gebannt.

Die konische Grenzfläche kann an sich beliebig gestaltet sein. Bevorzugt ist es, wenn der Spannkäfig einen konischen Außenrand aufweist. Er kann mit einer an dem vorderen Ende der Innenbohrung vorgesehenen Verengung zusammenwirken. Eine solche Verengung ist einfach in der Herstellung, da der übrige Teil der Innenbohrung im Wesentlichen zylindrisch ausgeführt sein kann. Es soll aber nicht ausgeschlossen sein, dass die Innenbohrung im Bereich des Passsitzes alternativ oder zusätzlich kegelig zulaufend ist.

Bei einer bevorzugten Variante ist der Passsitz zweizonig ausgeführt, wobei ein Segment an der Hülse und das andere an dem Spannkäfig angeordnet ist. Dabei ist das Segment an der Hülse das vordere Segment, und unmittelbar dahinter schließt sich das Segment am Spannkäfig an. Das hintere Segment am Spannkäfig hat seine größte Weite am Übergang zum vorderen Segment. Das hintere konische Segment schließt sich vorzugsweise glatt, d. h. ohne Weitensprung, an. Der Spannkäfig gemäß dieser bevorzugten Variante ist im hinteren Segment angeordnet und wirkt mit dem hohlkugelschalenartig geformten Segment derart zusammen, dass der Kugelkopf, wie vorstehend beschrieben, über den Äquator hinaus umfasst ist. Dabei geht bei der hier beschriebenen Variante der Spannkäfig selbst nicht über den Äquator hinaus. Diese zweiteilige Konstruktion ist montagefreundlich und sorgt für eine gute Sicherung des Kugelkopfs bei geringer Toleranzempfindlichkeit.

Der Spannkäfig weist an seiner hinteren Stirnfläche vorzugsweise eine rinnenartige Konkavität auf, die zur Aufnahme eines Haltestabs ausgebildet ist. Nähere Einzelheiten zu dem Haltestab werden weiter unten erläutert. Die Konkavität sorgt für einen Formschluss mit dem Haltestab und damit für eine positionsgenaue Halterung.

Der Spannkäfig ist zweckmäßigerweise in seinem hinteren Bereich seitlich geschlitzt, und zwar vorzugsweise als Doppelschlitz. Damit werden abspreizbare Zungen geschaffen. Sie sind zweckmäßigerweise so dimensioniert, dass sie in fensterartige Ausnehmungen an der Innenseite der Hülse eingreift. Damit ergibt sich eine zusätzliche Fixierung des Spannkäfigs mitsamt der von ihm gehalterten Knochenschraube, was einer unbeabsichtigten Depositionierung entgegenwirkt.

Vorzugsweise ist die Weite der Innenbohrung im hinteren Gewindebereich größer als die Weite im Bereich des Passsitzes. Dies ermöglicht eine einfache Vormontage des Spannkäfigs in der Haltehülse, indem nämlich der Spannkäfig von hinten durch den Gewindeabschnitt hindurch in seine Position im Bereich des Passsitzes eingebracht wird. Dies ermöglicht es, die Weite der vorderen Öffnung der Innenbohrung kleiner zu wählen als die Weite des Spannkäfigs. Damit ist der Spannkäfig gegenüber einem unbeabsichtigten Auswandern nach vorne gesichert.

Zweckmäßigerweise weist die Haltehülse einer Aufnahme für einen Haltestab eines Implantats auf. Damit kann eine sichere Fixierung des Implantats in die Bereiche des Knochens, in dem die Knochenschraube eingedreht ist, erreicht werden. Auch komplexe Implantate können auf diese Weise sicher und winkelstabil befestigt sein. Mit Vorteil ist die Aufnahme als eine Querbohrung zur Innenbohrung Haltehülse ausgeführt, und zwar im Bereich des Übergangs zwischen dem hinteren Innengewinde und dem vorderen Passsitz. Im eingesetzten Zustand befindet sich der Stab damit zwischen dem Druckelement und der Stirnseite des Spannkäfigs, auf welchen das Druckelement ohne den Stab unmittelbar einwirken würde. Bei eingesetztem Stab erfolgt die Druckübertragung vom Druckelement auf den Spannkäfig also mittelbar, nämlich über den Querschnitt des Stabs. Damit wird zugleich ohne weiteren Aufwand eine Fixierung des Stabs relativ zur Fixationsanordnung erreicht.

Die Querbohrung weist vorzugsweise einen unrunden Querschnitt auf. Damit kann eine formschlüssige Verbindung zwischen Stab und Haltehülse erreicht sein. Damit wird eine zusätzliche Sicherheit gegenüber unerwünschten Verdrehungen geschaffen. Dabei braucht die Querbohrung nicht unbedingt dieselbe Querschnittsform wie der Stab aufweisen, sondern zweckmäßigerweise ist die Querbohrung in Richtung der Längsachse der Innenbohrung länglich ausgedehnt. Dies bietet zusätzlichen Verstellraum und gewährleistet eine sichere Befestigung auch dann, wenn der Spannkäfig recht weit vorne positioniert ist (zum Ausgleich eines verhältnismäßig dünnen Kugelkopfs der Knochenschraube).

Vorzugsweise weist das Druckelement ein Sägezahngewinde auf, bei welchem die nach hinten weisende Lastflanke steiler ist als die nach vorne weisende andere Flanke. Besonders bewährt hat sich ein Lastflankenwinkel von 0° oder ein negativer Lastflankenwinkel.

Bei allen Ausführungsformen kann die Knochenschraube mit einer entlang ihrer Mittelachse verlaufenden Durchgangsbohrung versehen sein, d. h. sie ist kanüliert. Damit kann sie entlang eines durchgeschobenen Führungsdrahts eingesetzt werden, wodurch eine sehr gute Positionierungsgenauigkeit auch unter schwierigen Zugangsverhältnissen erreicht wird.

Die Erfindung wird nachfolgend in Bezugnahme auf die beigefügte Zeichnung erläutert, in der ein vorteilhaftes Ausführungsbeispiel dargestellt ist. Es zeigen:
- Fig. 1: eine Explosionsansicht der Fixationsanordnung gemäß einem ersten Ausführungsform der Erfindung;
- Fig. 2: eine Montageansicht mit eingesetztem Stab eines Implantats;
- Fig. 3: eine Querschnittansicht der Haltehülse der Fixationsanordnung;
- Fig. 4: eine Seitenansicht einer Fixationsanordnung gemäß einer zweiten Ausführungsform der Erfindung;
- Fig. 5: eine Detailvergrößerung aus Fig. 4;
- Fig. 6: perspektivische Ansichten von Spannhülsen;
- Fig. 7: Schnittdarstellungen von Spannhülsen;
- Fig. 8: verschiedene Ausführungsbeispiele für Verdickungen der Knochenschraube;
- Fig. 9: einen Mehrfachhalter für zwei Knochenschrauben;
- Fig. 10: eine Explosionsansicht der Fixationsanordnung gemäß einer Variante der ersten Ausführungsform;
- Fig. 11: eine Querschnittansicht der Haltehülse der Variante gemäß Fig. 10;
- Fig. 12: eine Darstellung einer Knochenschraube für eine Variante der zweiten Ausführungsform; und
- Fig. 13: eine Schnittdarstellung einer Spannhülse der Variante der zweiten Ausführungsform.

Die Fixationsanordnung umfasst einen Halter 2 für eine Knochenschraube 1. Die Knochenschraube 1 ist an sich herkömmlich ausgeführt mit einem Schaft 10, an dem in seinem vorderen Bereich oder vollständig ein Knochengewinde 11 vorgesehen ist. Am hinteren Ende des Schafts 10 ist ein Schraubenkopf 12 vorgesehen, der sphärisch ausgebildet ist mit einem Äquator 13 an seinem größten Durchmesser. An seinem hinteren Ende kann eine sternförmige Vertiefung 14 als eine Aufnahme für einen entsprechend ausgebildeten Schraubenschlüssel vorgesehen sein.

Der Halter 2 umfasst eine Hülse 3, einen Spannkäfig 4 sowie ein Druckelement 5. Die Hülse 3 ist hohlzylinderartig ausgeführt mit einer von einem hinteren zum vorderen Ende durchlaufenden Innenöffnung 30. Die Innenöffnung 30 weist in ihrem hinteren Bereich ein Innengewinde 31 und in ihrem vorderen Bereich einen mit glatter Wandung versehenen Passsitz 32 auf. Hierbei ist der Kerndurchmesser des Innengewindes 31 so gewählt, dass er ein wenig kleiner (etwa 1 mm) ist als der Durchmesser im Bereich des Passsitzes. Die Gestaltung der Wandung im Bereich des Passsitzes 32 ist im Wesentlichen zylindrisch mit einer Verengung 33 am vorderen Ende. Es wird so eine vordere Mündung mit einem verringerten Durchmesser gebildet. Die Hülse 2 weist weiter zwei diametral gegenüberliegende Längsschlitze 35 auf. Sie erstrecken sich vom hinteren Ende der Hülse 3 über den gesamten Bereich des Innengewindes 31 bis in den Übergangsbereich 34 zwischen Innengewinde 31 und Passsitz 32. Dort weist der Schlitz 35 in Tangentialrichtung eine Erweiterung auf.

Der Spannkäfig 4 weist einen mit einer konischen Außenfläche versehenen Mantel 40 auf. Dieser ist durch sechs sich vom vorderen Ende über etwa 4/5 der Länge des Spannkäfigs erstreckende Schlitze 41 in eine entsprechende Zahl von Segmenten 42 geteilt. Diese Segmente 42 sind über einen im Bereich der hinteren Stirnseite 43 gebildeten Haltering 45, der eine zentrale Montageöffnung 44 berandet, miteinander verbunden. An den vorderen Enden der Segmente 42 ist jeweils ein nach innen weisender Vorsprung 46 ausgebildet. Der Innendurchmesser des Spannkäfigs 4 ist dabei so gewählt, dass der Kugelkopf 12 der Schraube 1 darin mit geringem Spiel aufgenommen ist. Der Vorsprung 46 ragt soweit nach innen, dass die Segmente 42 nur unter elastischer Aufweitung über den Äquator 13 des Kugelkopfs 12 hinweg bewegt werden können. Damit ist der in den Spannkäfig 4 aufgenommene Kugelkopf 12 der Schraube 1 gegenüber einem unbeabsichtigten Herausfallen gesichert.

Das Druckelement 5 ist als eine Madenschraube ausgebildet mit einem Sägezahngewinde 51 an seinem Außenmantel. An der hinteren Stirnseite ist im Zentrum eine sternförmige Vertiefung 54 zur Aufnahme eines Schraubenschlüssels ausgebildet. Das Sägezahngewinde 51 ist so ausgeführt, dass die nach hinten weisende lasttragende Gewindefläche 52 senkrecht zur Mantelfläche ist, während die nach vorne weisende andere Gewindefläche 53 in einem Winkel von etwa 70° geneigt ist. Mit dieser asymmetrischen Sägezahn-Gestaltung des Gewindes 51 wird ein Aufweiten der Haltehülse 3 auch unter hoher Belastung vermieden.

Zur Montage wird der Käfig 4 unter elastischer Zusammenpressung der Segmente 42 von vorne durch die Verengung 33 geführt, bis der Spannkäfig 4 im Bereich des Passsitzes 32 zur Anlage kommt. Im eingesetzten Zustand ragen die vorderen Ende der Segmente 42 mit dem Vorsprung 46 aus der Verengung nach vorne heraus. Im nächsten Schritt wird die Schraube 1 mit ihrem Kugelkopf 12 in den Käfig 4 unter elastische Aufweitung der Segmente 42 eingesetzt. Damit sind diese Teile unverlierbar vormontiert. Hernach kann das Druckelement 5 einige Umdrehungen in das Innengewinde 31 eingesetzt sein. Dabei bleibt der vordere Teil des Schlitzes 35 mit der Erweiterung 36 frei. Die Fixationsanordnung ist nun einsatzbereit.

Zur Befestigung des Implantats wird zuerst die Knochenschraube 1 mittels eines durch die Hülse 3 zur sternförmigen Aufnahme 14 in den Kugelkopf 12 geführten Schraubenschlüssel festgezogen. Im nächsten Schritt kann zur Montage der Stab 7 in die Querbohrung 35 eingeführt werden. Schließlich wird das Druckelement 5 eingeschraubt und mit einem in die Vertiefung 54 eingesetzten Schraubenschlüssel festgezogen. Hierbei werden die erzeugten Druckkräfte über den Querschnittsstab 7 auf die Stirnseite 43 des Spannkäfigs 4 geleitet, wodurch dieser in Axialrichtung nach vorne gedrückt wird. Die hierbei entstandenen Druckkräfte werden aufgrund der Konizität im Bereich der Grenzfläche zwischen Außenmantel 40 des Spannkäfigs 4 und des Passsitzes 32 in Kombination mit der Verengung 33 in nach innen wirkende Presskräfte umgewandelt, die betragsmäßig um ein Vielfaches höher sind und den kugelförmigen Schraubenkopf 12 fest mit der Innenseite der Segmente 42 des Spannkäfigs 4 verpressen. Damit wird eine winkelstabile Fixation erreicht.

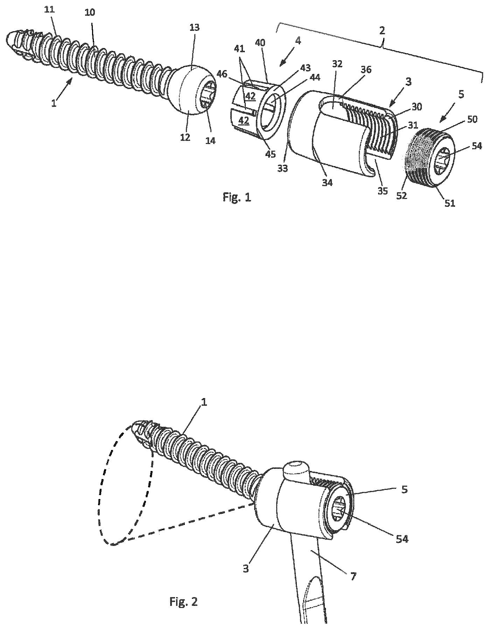

Die gestrichelte Linie in Figur 2 zeigt einen möglichen Bereich für die unterschiedlichen Winkel, welche die polyaxiale Lagerung der Schraube 1 in Bezug auf den Halter 3 einnehmen kann.

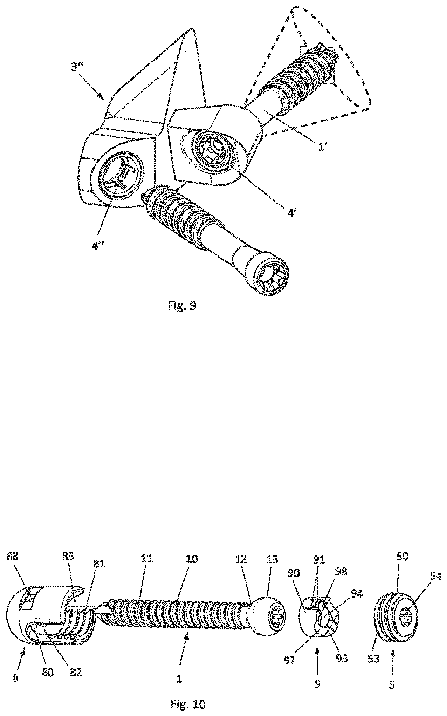

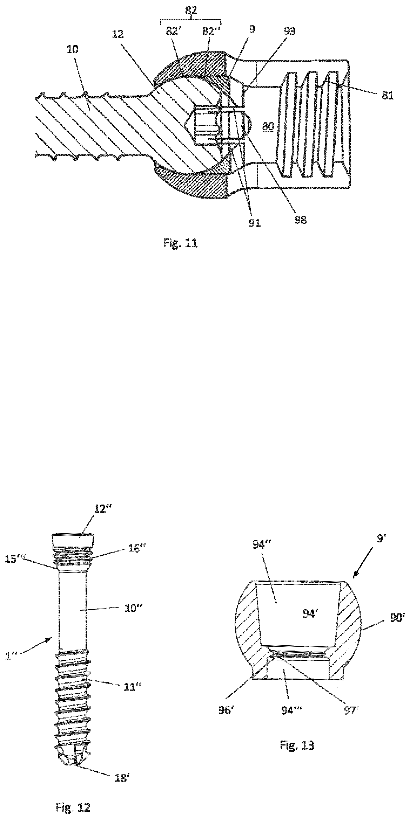

Eine Variante der ersten Ausführungsform ist in den Fig. 10 und 11 dargestellt. Gleichartige Teile tragen die gleichen Bezugsziffern. Der wesentliche Unterschied liegt in der Ausführung der Hülse 8 und des Spannkäfigs 9. Die Hülse 8 ist hohlzylinderartig ausgeführt mit einer von einem hinteren zum vorderen Ende durchlaufenden Innenbohrung 80, die in ihrem hinteren Bereich mit einem Innengewinde 81 zur Aufnahme des Druckelements 5 und in ihrem vorderen Bereich mit einem hohlkugelsegmentartig geformten Segment 82' eines Passsitzes 82 versehen. Im Zwischenbereich ist eine im wesentlichen glatte Innenwandung vorgesehen, an welche der Spannkäfig 9 mit seinem Mantel 90 anliegt. Das hohlkugelsegmentartig geformte Segment 82' des Passsitzes 82 ist komplementär zum Kugelkopf 12 der Knochenschraube 12 geformt. Die Mündung am vorderen Ende der Hülse 8 ist verengt und lässt den Schaft 10 passieren, hindert aber den Kugelkopf 12 an einem Durchgang.

Die Hülse 8 weist weiter zwei diametral gegenüberliegende Längsschlitze 85 auf. Sie erstrecken sich vom hinteren Ende der Hülse 8 über den gesamten Bereich des Innengewindes 81 bis zum Passsitz 82. Versetzt zu den Längsschlitzen 85 sind zwei fensterartige Ausnehmungen 88 angeordnet.

Der Spannkäfig 9 weist einen mit einer vorzugsweise konischen Außenfläche versehenen Mantel 90 sowie einen Innenraum 94 auf.

Dessen Weite ist auf die Weite des Kugelkopfs 12 derart abgestimmt, dass sich über einen Bereich eine Passung ergibt. Dieser Bereich bildet einen zweiten Teil 82'' des Passsitzes 82. Er wirkt mit dem hohlkugelartigen Teil 82' so zusammen, dass ein durchgehender Passsitz 82 zur Aufnahme des Kugelkopfs 12 gebildet ist. Die Fügestelle zwischen den beiden Teilen 82' und 82'' liegt im Bereich größter Weite und damit dort, wo der Äquator 13 der Knochenschraube liegt.

Im hinteren Bereich des Spannkäfigs sind Schlitzpaare 91 gegenüberliegend angeordnet, wodurch zwei diametral angeordnete Zungen 98 entstehen. Im eingesetzten Zustand des Spannkäfigs 9 spreizen sie ab und fassen in die fensterartigen Ausnehmungen 88, wodurch der Spannkäfig 9 an einem Herausfallen aus der Hülse 8 gehindert ist. Damit kann eine vormontierte Einheit aus in die Hülse 8 eingesteckter Knochenschraube mitsamt Spannkäfig 9 gebildet werden. An seiner hinteren Stirnfläche ist der Spannkäfig 9 versehen mit einer Rinne 97. Sie ist vorzugsweise halbzylindrisch geformt und dient zur formschlüssigen Aufnahme eines Haltestabs eines Implantats 7.

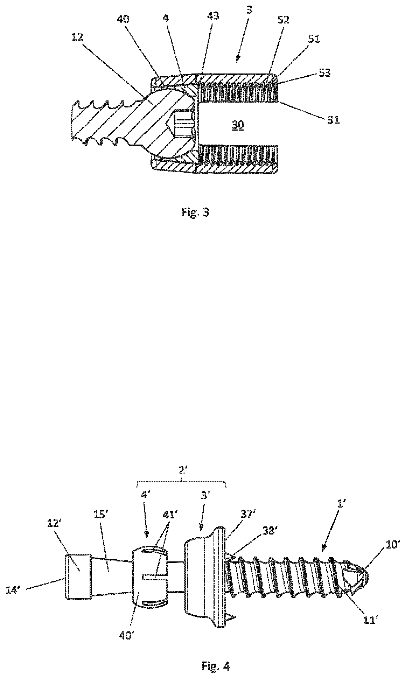

Es wird nun Bezug genommen auf die zweite Ausführungsform. Wie aus Fig. 4 zu erkennen ist, umfasst die Fixationsanordnung einen Halter 2' für eine Knochenschraube 1', die einen Schaft 10' mit einem im vorderen Bereich angeordneten Knochengewinde 11' aufweist. Zum hinteren Ende hin weist der Schaft 10' eine zunehmende Verdickung 15' auf. Sie erstreckt sich bis an einen Kopf 12', der eine Vertiefung 14' zur Aufnahme eines Schraubenschlüssels (nicht dargestellt) aufweist.

Der Halter 2' umfasst eine Grundplatte 3' und eine Spannhülse 4'. Die Grundplatte 3' weist an ihrer vorderen Seite eine Auflagefläche 37' zur Anlage an einem Knochen oder anderen Gewebeteilen auf. Sie kann mit optionalen Haltedornen 38' versehen sein. Die Grundplatte 3' ist durchquert von einer Halteöffnung 30', die in ihrem mittleren Bereich kugelkalottenförmig erweitert ist zu einem Passsitz 32'. In diesen ist die Spannhülse 4', welche ihrerseits einen sphärischen Mantel 40' mit passenden Abmessungen aufweist, aufgenommen. Durch die komplementären Flächen des sphärischen Mantels 40' einerseits und des kugelkalottenförmigen Passsitzes 32' andererseits ist die Spannhülse 4' schwenkbeweglich gelagert. In diesem Zustand ist die Fixationsanordnung vormontiert und bereit zur Implantation.

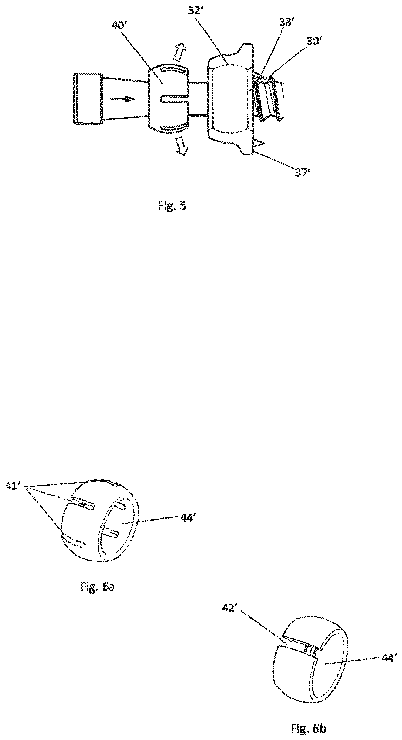

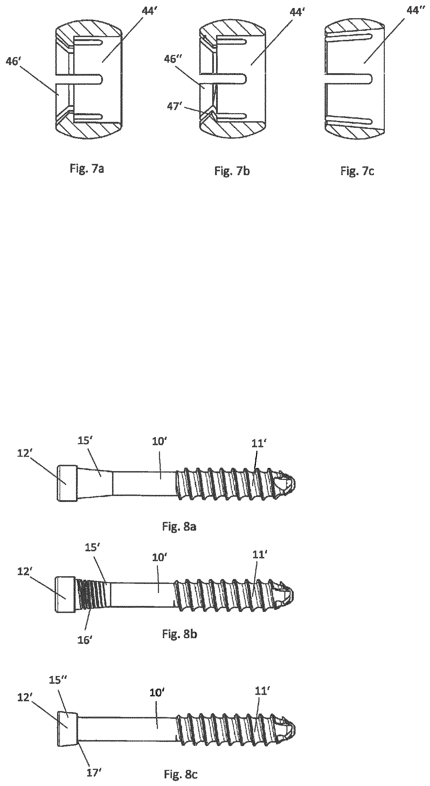

Die Spannhülse 4' weist eine zentrale Öffnung 44' auf, durch welche bei der Implantation die Knochenschraube 1' mit ihrem Schaft 10' gesteckt wird. Die Öffnung 44' weist eine im wesentlichen zylinderförmige Gestalt auf, mit einer nach innen weisenden Radialleiste 46' am vorderen Ende (s. Fig. 7a). Weitere Ausführungsbeispiele für die Öffnung 44', 44'' sind in Fig 7b und c dargestellt, nämlich mit einer komplex geformten Leiste 46'', an der ein oder zwei Umdrehungen eines Innengewindes 47' angeordnet sind, oder eine leistenlose Ausführung mit konisch sich nach vorne verengender Öffnung. Die Öffnung kann alternativ aber auch zylindrisch sein, wenn der Schaft der Knochenschraube konisch ausgeführt ist. Die Spannhülse 4' ist vorzugsweise geschlitzt, und zwar mit mehreren einseitig nach vorne offenen Schlitzen 41' (s. Fig. 6a). Es kann aber auch ein durchgehender Schlitz 42' vorgesehen sein (s. Fig. 6b) .

Die Verdickung 15' erstreckt sich über einen Bereich, der etwa zwei bis dreimal so lang ist wie die durch die Länge der Öffnung 44' bestimmte Länge der Spannhülse 4'. Beim Eindrehen der Schraube 1' bewegt sich diese axial nach vorne mit ihrem Schaft 10' durch die Grundplatte 3' (symbolisiert durch den Einfachpfeil in Fig. 5). Dabei kommt die konische Verdickung 15' in Kontakt mit der Innenwandung der Öffnung 44', und spreizt bei der weiteren Axialbewegung die Spannhülse auf (s. Doppelpfeil in Fig. 5). Dadurch wird die Spannhülse 4' mit ihrem sphärischen Mantel 40' gegen den kugelkalottenförmigen Passsitz 32 gezwängt. Hierbei entstehen wegen des geringen Konuswinkels von weniger als 10 Grad aufgrund der Keilwirkung erhebliche Kräfte, so dass sich effektiv eine Verpressung zwischen Spannhülse 4' und dem Passsitz 32' der Grundplatte 3' ergibt. Die Schraube 1' ist damit in ihrer jeweiliger verschwenkten Winkellage relativ zu der Grundplatte 3' fixiert.

Die Verdickung 15' kann, wie vorstehend beschrieben, konisch sein. Dies ist in Fig. 8a dargestellt. Alternative Ausführungen sind in Fig. 8b und c dargestellt. So kann die Verdickung 15' mit einem Sekundärgewinde 16' versehen sein. Dieses ist mehrgängig zum einfacheren Einsuchen in das Innengewinde 47' an der Spannhülse 4' und weist vorzugsweise dieselbe Steigung wie das Gewinde 11' am Schaft 10' auf. Damit kann die Axialkraft und damit die Spreiz- und Presswirkung erhöht werden. Außerdem verbessert sich der Schutz gegen ein unbeabsichtigtes Lösen durch Rückdrehen der Schraube 1' im Knochen. Bei einer weiteren alternativen Ausführung ist die Verdickung 15'' weiter zum hinteren Ende verschoben und weist eine nach vorne weisende Bundfläche 17' auf. Der konische Bereich der Verdickung kann damit kürzer ausgeführt sein, so dass eine bauliche Integration mit dem Kopf 12' ermöglicht ist. Das ergibt eine besonders einfach herzustellende Knochenschraube 1'. Vorzugsweise wird die letzte Alternative verwendet zusammen mit einer Art von Spannhülse 4', wie sie in Fig. 7c dargestellt ist. Sie ermöglicht dank ihrer gleichfalls konischen Öffnung 44'', die vorzugsweise komplementär zu der Konizität der Verdickung 15'' ist, eine besonderes kompakt bauende Einheit.

Eine Variante der zweiten Ausführungsform ist in Fig. 12 und 13 dargestellt. Gleichartige Teile sind mit gleichen Bezugsnummern versehen. Die Knochenschraube 1'' weist einen Schaft 10'' mit einem im vorderen Bereich angeordneten konischen Knochengewinde 11'' auf. Am hinteren Ende weist der Schaft 10'' eine Verdickung 15''' auf, an die sich der Kopf 12'' anschließt. Der Kopf 12'' ist ausgeführt, dass er sich ausgehend von der Verdickung 15''' nach hinten konisch erweitert. Die Verdickung 15''' umfasst einen konischen vorderen Abschnitt im Bereich des Übergangs zum Schaft 10'' und einen Mantel im hinteren Abschnitt, an dem ein Sekundärgewinde 16'' ausgebildet ist. Es weist eine Steigung auf, die etwas weniger als die Hälfte der Steigung des Knochengewindes 11'' betragt (beispielsweise 7/15).

Eine Variante der Spannhülse 9' ist in Fig. 13 dargestellt. Sie unterscheidet sich von den in Fig. 7 dargestellten Spannhülsen 4' im wesentlichen durch eine komplexe Gestaltung der Durchgangsöffnung 94'. Diese umfasst einen kürzeren vorderen zylindrischen Teil 94''' und einen längeren hinteren konischen Teil 94'', wobei an deren Übergang eine nach innen ragende Radialleiste 96' mit einem kurzen Innengewinde 97' angeordnet ist. Unter "kurz" werden hierbei bis zu zwei Umdrehungen verstanden.

Bei dieser Variante weitet die Knochenschraube 1'' mit ihrer Verdickung 15''' den zylindrischen Teil 94''' der komplexen Durchgangsöffnung, und zwar unter einer Vorschubkraft, die durch das in dem kurzen Innengewinde 97' eingreifende Sekundärgewinde 16'' beim Einschrauben erzeugt wird. Die konische Form des Kopfs 12'' sorgt zusammen mit dem konischen Teil 94'' für eine weitere Aufspreizung und Verklemmung der Knochenschraube 1'', so dass sie gegen ein unbeabsichtigtes Lösen gesichert ist.

Der Halter kann auch so ausgebildet sein, dass mehrere Knochenschrauben gehaltert sind. Ein Beispiel hierfür ist in Fig. 9 dargestellt. Dargestellt ist ein Mehrfachhalter 3'', der zwei Aufnahmesitze aufweist, um zusätzlich noch eine zweite Spannhülse 4'' aufzunehmen.

## Patentansprüche

1. Fixationsanordnung für ein Implantat mit einer Schraube (1) und einem Halter (2), wobei die Schraube (1) einen Gewindeschaft (10) und einen Kugelkopf (12) aufweist, und der Halter (2) eine Haltehülse (3, 8), ein Spannelement (4, 9) und ein Druckelement (5) umfasst, wobei die Haltehülse (3, 8) eine durchlaufende Innenbohrung (30, 80) mit einem vorderen Passsitz (32, 82) für das Spannelement und einem hinteren Innengewinde (31, 81) für das Druckelement (5) versehen ist,
**dadurch gekennzeichnet, dass**
das Spannelement als Spannkäfig (4, 9) ausgeführt ist und im eingesetzten Zustand der Kugelkopf (12) über dessen Bereich größter Weite (13) hinaus umschlossen ist und über eine Grenzfläche (40, 90) so mit dem Passsitz (32, 82) zusammenwirkt, dass der Spannkäfig (4, 9) bei einer axialen Verschiebung nach vorne zusammengedrückt und gegen den Kugelkopf (12) verpresst ist.

2. Fixationsanordnung nach Anspruch 1, **dadurch gekennzeichnet, dass** das Druckelement (5) auf einen Außenbereich (45) einer Stirnseite (43, 93) des Spannkäfigs (4, 9) wirkt.

3. Fixationsanordnung nach Anspruch 1 oder 2, **dadurch gekennzeichnet, dass** die Weite einer vorderen Mündung der Innenbohrung (30, 80) kleiner ist als die Weite des Spannkäfigs (4, 9).

4. Fixationsanordnung nach einem der Ansprüche 1 bis 3,
**dadurch gekennzeichnet, dass** der Spannkäfig (4, 9) einen konischen Außenmantel (40, 90) aufweist.

5. Fixationsanordnung nach einem der Ansprüche 1 bis 4,
**dadurch gekennzeichnet, dass** der Spannkäfig (4) vorzugsweise zum vorderen Ende offene Schlitze (41) aufweist.

6. Fixationsanordnung nach einem der Ansprüche 1 bis 5,
**dadurch gekennzeichnet, dass** der Spannkäfig (4) an seinem vorderen Ende einen radial vorspringenden Rand (46) aufweist.

7. Fixationsanordnung nach einem der Ansprüche 1 bis 6,
**dadurch gekennzeichnet, dass** die Weite der Innenbohrung (30) im Bereich des Innengewindes (31) kleiner ist als die Weite im Bereich des Passsitzes (32).

8. Fixationsanordnung nach einem der Ansprüche 1 bis 4,
**dadurch gekennzeichnet, dass** der Passsitz (82) aus mindestens zwei Segmenten (82', 82'') gebildet ist, von denen eines an der Hülse (8) und das andere an dem Spannkäfig (9) angeordnet ist.

9. Fixationsanordnung nach Anspruch 8, **dadurch gekennzeichnet, dass** das Segment (82'') an dem Spannkäfig (9) seine größte Weite dort hat, wo sich das andere Segment (81') anschließt.

10. Fixationsanordnung nach einem der Ansprüche 1 bis 4,
**dadurch gekennzeichnet, dass** der Spannkäfig (9) in seinem hinteren Bereich abspreizbare Zungen (98) aufweist.

11. Fixationsanordnung nach Anspruch 10, **dadurch gekennzeichnet, dass** die Hülse (8) in ihrem vorderen Bereich fensterartige Ausnehmungen (88) aufweist, in welche die abspreizbaren Zungen (98) im montierten Zustand des Spannkäfigs (98) eingreifen.

12. Fixationsanordnung nach einem der Ansprüche 1 bis 4,
**dadurch gekennzeichnet, dass** der Spannkäfig (9) an seiner hinteren Stirnfläche (93) eine rinnenartige Konkavität (97) aufweist.

13. Fixationsanordnung nach einem der Ansprüche 1 bis 12,
**dadurch gekennzeichnet, dass** die Haltehülse (3, 8) eine Aufnahme (35, 85) für einen Haltestab eines Implantats (7) umfasst.

14. Fixationsanordnung nach Anspruch 13, **dadurch gekennzeichnet, dass** die Aufnahme (35, 85) als eine Querbohrung zur Innenbohrung (30, 80) im Bereich des Übergangs zwischen dem Gewinde (31, 81) und Passsitz (32, 82) angeordnet ist.

15. Fixationsanordnung nach Anspruch 13 oder 14, **dadurch gekennzeichnet, dass** die Aufnahme (35, 85) einen unrunden Querschnitt aufweist.

16. Fixationsanordnung nach einem der Ansprüche 13 bis 15,
**dadurch gekennzeichnet, dass** die Querbohrung (35, 85) in Richtung einer Längsachse der Haltehülse (3, 8) länglich ausgedehnt ist.

17. Fixationsanordnung nach einem der Ansprüche 13 bis 16, **dadurch gekennzeichnet, dass** das Druckelement (5) ein Sägezahngewinde (51) aufweist, bei dem die nach hinten weisende Lastflanke des Gewindes (51) steiler ist als die nach vorne weisende Flanke (52).

18. Fixationsanordnung nach einem der vorangehenden Ansprüche, **dadurch gekennzeichnet, dass** die Knochenschraube (1, 1', 1'') kanüliert (18') ist.
